# EUROPEAN PATENT APPLICATION

(11) **EP 1 602 663 A1**
(43) Date of publication of application: **07.12.2005**
(21) Application number: 04076662.8
(22) Date of filing: 04.06.2004
(51) Int. Cl.: C07K 9/00, C07K 1/107, C07D 249/04

(54) **Triazole-linked glycoamino acids and glycopeptides**

(71) Applicant: Chiralix B.V., 6525 EC Nijmegen (NL)
(72) Inventor: Kuijpers, Brian Hubert Margaretha, 6533 SB Nijmegen (NL); Groothuys, Stan, 6533 VZ Nijmegen (NL); Van Delft, Floris Louis, 6523 KV Nijmegen (NL); Rutjes, Floris Petrus Johannes Theodorus, 6605 ZS Wijchen (NL); Blaauw, Richard H., 1092 EH Amsterdam (NL)
(74) Representative: van Westenbrugge, Andries

(57) **Abstract**

The invention concerns stable analogues of glycoamino acids and glycopeptides in which the non-sugar acetal heteroatom is replaced by a triazole moiety, e.g.
compounds of formula I or I' 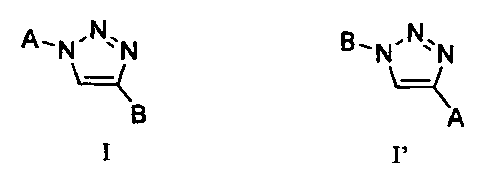 wherein A represents a carbohydrate which is coupled via its anomeric center to the triazole ring and B represents an amino acid residue.

## Description

### FIELD OF THE INVENTION

The present invention concerns stable analogues of glyco-amino acids and glycopeptides. The compounds of the invention are of use in for example medical applications, nutritional applications as well as research tools.

### BACKGROUND OF THE INVENTION

Glycoproteins are widely distributed in all forms of life, with the possible exception of the eubacteria. They occur in cells, both in soluble and membrane-bound forms, as well as in the extracellular matrix and in extracellular fluids. The most common glycoproteins are those in which the carbohydrate is linked to the protein by glycosyl linkages. Glycosylation represents one of the important co-translational and post-translational modifications of proteins.

In naturally occurring glycoproteins and in glycopeptide hormones, carbohydrate moieties play key roles in intercellular and intracellular transport of gene products (exit passport hypothesis), as well as extending the biological half-life of the active peptides in vivo (proteolytic protection). Additional roles supported by experimental evidence include the alteration of peptide backbone conformation (protein folding), control of membrane permeability, and molecular recognition (the concept of carbohydrate "antennae").

Thus it becomes increasingly more apparent that glycoproteins and -peptides play important roles in organisms. For example abnormalities in O-linked glycopeptides are implicated in numerous disease states. Abnormal post-translational modification of the tau protein has been implicated in the formation of neurofibrillary tangles of Alzheimer's disease. The antigenic T-epitopes and TN-epitopes of cell-surface glycopeptides have long been associated with cancer and used as tumor cell markers.

Therefore there is a need for glycopeptides that can be of use as research tools, but also for glycopeptides that can be of use in medicaments in case of glycopeptide related disorders. The chemical synthesis of glycopeptides provides an important tool for the study of glycopeptide hormones, glycoproteins and other complex carbohydrate structures found at the cell surface and in the glycocalyx. However, the synthesis and potential applications of glycopeptides are complicated due to the sensitivity toward chemical and enzymatic hydrolysis of the glycosidic bond linking the carbohydrate to the peptide. What is needed in the art are stable analogues of glycopeptides that are readily accessible preferably via chemical synthesis. This can be translated in a need for stable analogues of amino acid glycosides, hereinbelow also referred to as glyco-amino acid, which can be used to make glycopeptides.

### SUMMARY OF THE INVENTION

The present invention provides stable analogues of glyco-amino acids by compounds of the following formula I and I' 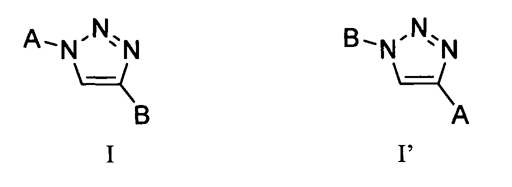 wherein A represents a carbohydrate which is coupled via its anomeric center (I) or parent anomeric center (I') to the triazole ring and B represents an amino acid or a mimetic of an amino acid

In a further aspect the present invention provides stable analogues of glycopeptides by compounds of the following formula II and II' 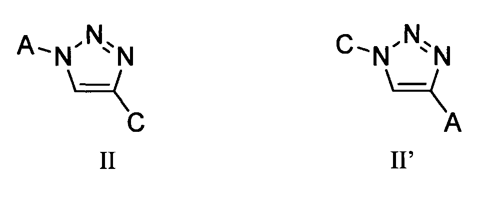 wherein A represents a carbohydrate which is coupled via its anomeric center (II) or parent anomeric center (II') to the triazole ring and C represents a polypeptide or a mimetic of a polypeptide.

In one embodiment the invention concerns compounds I and/or II. In one embodiment the invention concerns compounds I' and/or II'.

In the context of this invention the anomeric center means the central position (atom) in a (hemi)acetal, and a parent anomeric center is the carbon atom that was the central position (atom) in a (hemi)acetal in the parent carbohydrate.

### DETAILED DESCRIPTION OF THE INVENTION

In prominent classes of naturally occurring glycoproteins and glycopeptides the hemiacetal group of a carbohydrate is linked to an amino acid in a polypeptide via an alcohol functional group or amine functional group in the amino acid resulting in an O-glycosidic bond (O,O-acetal) or N-glycosidic bond (N,O-acetal). The present invention is based on the surprising insight that such a type of linkage can be rendered stable by replacing the non-sugar acetal heteroatom by a triazole moiety. The resulting carbohydrate-triazole-amino acid or -(poly)peptide compound can be readily synthesised under mild conditions and proved to be surprisingly stable, in particular stable compared to the otherwise readily hydrolysable glycosidic bond linking carbohydrate and peptide, and is suitable for a variety of applications.

In the context of this invention a glycopeptide is a compound containing a carbohydrate (or glycan) covalently linked via its (parent) anomeric center to a peptide (or mimetic) composed of amino acids (or mimetics). The carbohydrate may be in the form of a protected or unprotected monosaccharide, disaccharide(s), oligosaccharide(s), polysaccharide(s), or their derivatives (e.g. sulfo- or phospho-substituted). One, a few, or many carbohydrate units may be present.

From literature compounds having a triazole linked to the anomeric center of a carbohydrate are known, see De Las Heras et al. J.Med.Chem. (1979), 22, 496-500 and Harmon et al. J.Chem.Soc.D (1971) 296. However, these compounds are scarce and limited in diversity, which is probably due to the harsh conditions needed for their formation. This has limited the applicability of this compound class over the years. Recently, novel mild metal-catalysed procedures for the formation of triazoles have been reported. However, these procedures employ Lewis acid catalysts, which in the light of the fact that benzotriazoles connected to acetalic carbons can readily act as a leaving group under mild (Lewis)acidic conditions (see for example Veerman et al. Eur.J.Org.Chem. (2002) 18, 3133-3139, Deniau et al. Tetrahedron Lett. (2002) 43, 8055-8058 and Katrizky et al. J.Org.Chem. (2002) 67, 8239-8242). The combination of this property and a reactive anomeric carbon atom was not expected to be viable. Yet further, in figure 7 of WO 03/101972 a compound is depicted that comprises carbohydrate units to which a substituted triazole is linked to the 2-deoxy position of the carbohydrate. In Tornøe et al., J.Org.Chem. (2002) 67, 3057-3064 peptidotriazoles linked to 2-deoxygalactose (compounds 3r and 8b) have been described. Similarly substituted triazoles linked to the anomeric carbon in carbohydrates however are strikingly absent in the art.

A peptide in the context of this invention may either be an oligopeptide, comprising 2-10 amino acids, or a polypeptide, comprising more than 10 amino acids. The amino acids in the compounds of the invention and the amino acids in a peptide in the compounds of the invention may be D- or L-amino acids and include non-proteogenic amino acids. A mimetic of an amino acid is a compound that mimics the structure of an amino acid and that is suitable to be incorporated into an oligomer or polymer of amino acids thereby forming a peptide comprising a single mimetics of an amino acid or multiple mimetics of amino acids or consisting substantially in total of mimetics of amino acids. The latter is referred to as a mimetic of a polypeptide. In the context of this invention mimetics of amino acids are for instance compounds selected from the group consisting of β2 amino acids, β3 amino acids, α,α-disubstituted amino acids, β-amino sulfonyl compounds, α-aminohydrazino acids, α-hydroxy acids, α-amino nitriles. In one embodiment C in structure II or II' comprises 2-100 amino acids, preferably 2-50, more preferably 2-20 or preferably 10-100, more preferably 10-50, even more preferably 10-30 amino acids.

A glyco-amino-acid is a carbohydrate attached via its (parent) anomeric center to a single amino acid by any kind of covalent bond. Commonly occurring glyco-amino-acids are (see Sharon, N. & Lis, H. (1982) in The proteins, 3rd edn (Neurath, H. & Hill, R. L., eds) vol. 5, pp. 1-144, Academic Press, New York) β-*N*-acetylglucosaminylasparagine ((GlcNAc-)Asn) which is widely distributed in animals, plants and micro organisms, α-*N*-acetylgalactosaminylserine or -threonine ((Gal-NAc)Ser, (GalNAc-)Thr) which occur in glycoproteins of animal sources, β-xylosylserine ((Xyl-)Ser) occurring in proteoglycans, human thyroglobulin, β-galactosylhydroxylysine ((Gal-)Hyl) occurring in collagens and β-L-arabinosylhydroxyproline ((L-Ara-)Hyp) which occur in plant and algal glycoproteins. In the context of this invention a glyco-amino acid is a carbohydrate linked to an amino acid via a triazole moiety. According to the invention the (parent) anomeric carbon atom of the carbohydrate is linked to the triazole moiety.

The amino acid may be linked via its amine group, via its acid group or via its side chain. In a preferred embodiment the amino acid is linked to the triazole moiety via its side chain, leaving the amine group and the acid group intact and free to form amide bonds in a peptide chain.

In the context of this invention the generic term 'carbohydrate' includes monosaccharides, disaccharides, oligosaccharides and polysaccharides as well as substances derived from monosaccharides by reduction of the carbonyl group (alditols), by oxidation of one or more terminal groups to carboxylic acids, or by replacement of one or more hydroxy group(s) by a hydrogen atom, an amino group, a thiol group or similar heteroatomic groups. It also includes derivatives of these compounds. The term 'sugar' is frequently applied to monosaccharides and lower oligosaccharides.

Parent monosaccharides are polyhydroxy aldehydes H-[CHOH]ₙ-CHO or polyhydroxy ketones H-[CHOH]ₙ-CO-[CHOH]ₘ-H with four or more carbon atoms. The generic term 'monosaccharide' (as opposed to oligosaccharide or polysaccharide) denotes a single unit, without glycosidic connection to other such units. It includes aldoses, dialdoses, aldoketoses, ketoses and diketoses, as well as deoxy sugars and amino sugars, and their derivatives, provided that the parent compound has a (potential) carbonyl group.

The carbohydrate in the compounds of the invention may be similar to that occurring in natural glycoproteins and peptides, which are comparatively small, optionally branched, usually unsulfated carbohydrate units, often devoid of repeating units. In one embodiment the carbohydrate is in the form of oligosaccharides, linear or branched, containing up to about 20 monosaccharide residues; in a further embodiment the compounds of the invention contain linear mono-, di-, tri-, tetra-, penta-, hexa-, hepta-, octa-, nona-, or decasaccharide units, preferably mono-, di-, tri- or tetrasaccharide units, more preferably tetrasaccharide units; in yet a further embodiment the carbohydrates may contain or consist of repeating units, such as for instance repeating units of *N*-acetyllactosamine.

A preferred method to prepare the compounds of the invention is via the cycloaddition of azides and alkynes to give triazoles. This reaction is also known as the Huisgen 1,3-dipolar cycloaddition, see R. Huisgen, Pure Appl. Chem. 1989,61, 613-628; R. Huisgen et al., Chem. Ber. 1967, 100, 2494-2507; W. Lwowski, in 1,3-Dipolar Cycloaddition Chemistry, (Ed. A. Padwa), Wiley, New York, 1984; vol. 1, Chapter 5. The particular usefulness of this cycloaddition has been overlooked for quite some time until it recently gained renewed interest, see A.R. Katritzky, Y.M. Zhang, S.K. Siingh *Heterocycles,* **2003**, *60*, 1225-1239; W. S. Home, C. D. Stout, M R. Ghadiri *J. Am. Chem. Soc*. **2003,** *125,* 9372-9376; E. Speers, G. C. Adam, B. F. Cravett *J. Am. Chem. Soc*. **2003,** *125,* 4686-4687; S. Löber, P. Rodriguez-Loaiza, P. Gmeiner *Org. Lett*. **2003**, *5*, 1753-1755; S. Kamijo, T. Jin, Z. Huo, Y. Yamamoto *J. Am. Chem. Soc*. **2003,** *125,* 7786-7787; L. V. Lee, M. L. Mitchell, V. V. Fokin, K.B. Sharpless, C-H. Wong J. *Am. Chem. Soc*. **2003,** *125,* 9588-9589; Q. Wang, T. R. Chan, V. V. Fokin, K. B. Sharpless, M. G. Finn *J. Am. Chem. Soc*. **2003**, *125*, 3192-3193; T. S. Seo, Z. Li, H. Ruparel, J. Ju *J*. *Org. Chem.* **2003,** *68*, 609-612; V.V. Rostovtsev, L. G. Green, V. V. Fokin, K. B. Sharpless *Angew. Chem. Int. Ed*. **2002**, *41*, 2596-2599; S. Borman, C & EN *Chemical & Engineering News* **2002,** *80, 29-34;* Z. P. Demko, K. B. Sharpless *Angew. Chem. Int. Ed*. **2002,** *41*, 2213-2116; Z. P. Demko, K. B. Sharpless *Angew. Chem. Int. Ed*. **2002**, *41*, 2210-2113; W. G. Lewis, L. G. Green, M. G. Finn, K. B. Sharpless *Angew. Chem. Int. Ed.* **2002**, *41*, 1053-1057; W. Tornøe, C. Christensen, M. Meldal *J*. *Org. Chem.* **2002,** *67,* 3057-3064 and H. C. Kolb, M. G. Finn, K. B. Sharpless *Angew. Chem. Int. Ed.* **2001,** *40,* 2004-2021.

To prepare the novel glyco-amino-acids and glycopeptides the following cycloaddition reaction partners are suitable: a carbohydrate bearing an azide group and an amino acid or peptide bearing an acetylene group or alternatively a carbohydrate bearing an acetylene group and an amino acid or peptide bearing an azide group. Methods for the preparation of each of these cycloaddition partners are well known in the art and are described for instance in Shiozaki et al. *Chem. Lett*., **1996,** *9*, 735-736; Horton, D. *Methods in Carbohydrate Chemistry*; Whistler, R. L., BeMiller, J. N., Eds. Academic Press: New York, 1972; Vol. VI, pp 282-285; Lehnhoff et al. *Angew.Chem.* **1995**, *107*, 1208-1211.

Suitable amino acids bearing an acetylene group are for example: 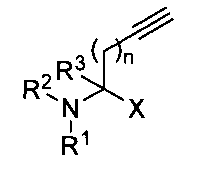 wherein X represents CN, CO₂R⁴, COR⁵, SO₂R⁶, CH₂OR⁷, CH₂NR⁸R⁹, CR¹⁰R¹¹, CONR¹²NR¹³R¹⁴;
R¹, R², R³, R⁴, R⁵, R⁶, R⁷, R⁸, R⁹, R¹⁰, R¹¹, R¹², R¹³ and R¹⁴ may be the same or different, and each independently represent H, linear or branched and optionally substituted alkyl, optionally substituted acyl, optionally substituted sulfonyl, optionally substituted aryl, and amine and/or acid protective groups, n = 1-10.

Suitable amino acids bearing an azide group are for example: 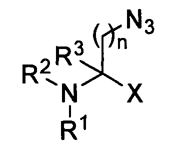 wherein X represents CN, CO₂R⁴, COR⁵, SO₂R⁶, CH₂OR⁷, CH₂NR⁸R⁹, CR¹⁰R¹¹, CONR¹²NR¹³R¹⁴;
R¹, R², R³, R⁴, R⁵, R⁶, R⁷, R⁸, R⁹, R¹⁰, R¹¹, R¹², R¹³ and R¹⁴ may be the same or different, and each independently represent H, linear or branched and optionally substituted alkyl, optionally substituted acyl, optionally substituted sulfonyl, optionally substituted aryl, and amine and/or acid protective groups, n =1-10. and 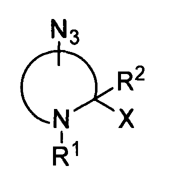 wherein X represents CN, CO₂R³, COR⁴, SO₂R⁵, CH₂OR⁶, CH₂NR⁷R⁸, CR⁹R¹⁰, CONR¹¹NR¹²R¹³;
R¹, R², R³, R⁴, R⁵, R⁶, R⁷, R⁸, R⁹, R¹⁰, R¹¹, R¹² and R¹³ may be the same or different, and each independently represent H, linear or branched and optionally substituted alkyl, optionally substituted acyl, optionally substituted sulfonyl, optionally substituted aryl, and amine and/or acid protective groups, the ring may substituted and be saturated or (partly) unsaturated and may be 4-10 membered; the azide can be in any position on the ring.

The amino acids may also be part of a peptide, in which case in the structures given above X may represent the site of attachment to one or more further amino acids, and/or R¹ and/or R² may also represent the site of attachment to one or more further amino acids.

The amino acids may be in an optically active form, and can be either in the R or S configuration at any stereocenter.

The amino group and/or the acid group in the amino acids may be suitably protected in order to facilitate further reaction steps.

Preferably the amino acid bears the azide or acetylene group in the side chain leaving for instance the amine and/or acid functionalities intact and free to form amide bonds in a peptide chain.

Of particular interest are the amino acids bearing azide or acetylene groups that closely resemble the amino acids in naturally occurring glycopeptides that are linked to carbohydrates. Thus in particular serine, threonine, lysine, hydroxylysine, asparagine and hydroxyproline that bear an azide or acetylene group, or in which, compared to the natural amino acid, an OH or NH₂ functional group is modified into an azide or acetylene group are of interest.

Any saccharide unit in a carbohydrate as described above may contain either an azide or acetylene group at the (parent) anomeric position. Preferably the azide or acetylene is in a terminal saccharide unit at the (parent) anomeric position of the saccharide unit.

Thus, suitable carbohydrates bearing an acetylene group are: 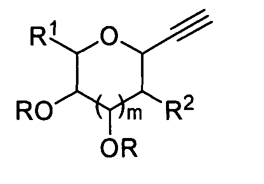 wherein R¹ may be H, CH₂OR or CHORCH₂OR, R² may be H, NHR or OR, R may be the same or different within one molecule and may be H, a saccharide unit or a protective group, m = 0 or 1.

Suitable carbohydrates bearing an azide group are: 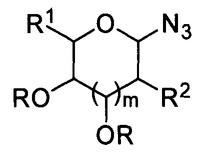 wherein R¹ may be H, CH₂OR or CHORCH₂OR, R² may be H, NHR or OR, R may be the same or different within one molecule and may be H, a saccharide unit or a protective group, m = 0 or 1.

Thus, suitable carbohydrates bearing an acetylene or azide on the anomeric carbon are derived from allo-, altro-, gluco-, manno-, gulo-, ido-, galacto-, talo-, ribo-, arabino-, xylo- and lyxofuranoside and from allo-, altro-, gluco-, manno-, gulo-, ido-, galacto-, talo-, ribo-, arabino-, xylo- and lyxopyranoside.

The carbohydrates can be either in the D or L configuration.

The cycloaddition of the azide and acetylene reaction partners can proceed according to any of the methods disclosed in the literature cited above. A series of advantageous conditions for the cycloaddition reaction is also disclosed in WO 03/101972. The cycloaddition results in substituted-[1,2,3]-triazoles. The cycloaddition may result in two possible regioisomers, *viz*. the 1,4- and 1,5-substituted triazole. The 1,4-substituted product is preferred, which also will be the most abundant, if not sole, regioisomer under standard cycloaddition conditions.

In one embodiment the invention concerns a method for the preparation of a glyco-amino acid or glycopeptide by reacting a carbohydrate having an azide group on the anomeric carbon as described above with an amino acid or peptide comprising an amino acid having an acetylene group as described above.

In one embodiment the invention concerns a method for the preparation of a glyco-amino acid or glycopeptide by reacting a carbohydrate having an acetylene group on the (parent) anomeric carbon as described above with an amino acid or peptide comprising an amino acid having an azide group as described above.

Cycloaddition of an azide or acetylene bearing amino acid with a carbohydrate reaction partner, respectively an acetylene or azide bearing carbohydrate, results in a glyco-amino-acid of the invention.

Such a glyco-amino acid may be used as a building block in the preparation of a glycopeptide according to the invention. For instance one or more of such glyco-amino acids may be used in an automated peptide synthesiser. Alternatively one or more acetylene bearing amino acids, or azide bearing amino acids may be used as building blocks for the preparation of a peptide. During the peptide synthesis, as the chain of amino acids increases, or after the synthesis of a desired peptide has been completed, the peptide having azide or acetylene functional groups may be reacted with a carbohydrate cycloaddition reaction partner in order to obtain a glycopeptide according to the invention.

Thus in an embodiment the invention relates to a method for the preparation of a glycopeptide of the invention, said method comprising reacting a glyco-amino acid of the invention as a building block in a peptide synthesis, such as a peptide synthesis on a solid support, preferably in an automated peptide synthesiser.

In another embodiment the invention relates to a method for the preparation of a glycopeptide of the invention, said method comprising reacting an azide bearing amino acid as described above and/or an acetylene bearing amino acid as described above as a building block in a peptide synthesis, such as a peptide synthesis on a solid support, preferably in an automated peptide synthesiser, and reacting azide functional groups in the resulting peptide during peptide synthesis or after peptide synthesis is completed with a carbohydrate having an acetylene group on the (parent) anomeric carbon as described above and/or reacting acetylene functional groups in the resulting peptide during peptide synthesis or after peptide synthesis is completed with a carbohydrate having an azide group on the anomeric carbon as described above.

Thus a glycopeptide of the invention may comprise one, two, three, four or more carbohydrates attached to different amino acids in the peptide chain. The glyco-amino acid can be at any position in the peptide chain.

The methods described above may require appropriate protective group manipulations on carbohydrate, amino acid and/or peptide. Such protective group manipulations are well known in the art and suitable protective groups can be found in e.g. Greene, Protective Groups in Organic Synthesis, 3^{rd} edition 1999, Wiley-Interscience, New York.

Glycopeptides may be used for the treatment of bacterial infections. Therefore, in another embodiment, the present invention is directed to a method for controlling a bacterial infection in a host animal, typically a warm-blooded animal, which comprises administering to the host animal an effective, antibacterial amount of a compound of the present invention. In this embodiment, the compounds can be used to control and treat infections due to various bacteria, but especially gram-positive bacteria. In a preferred embodiment, the compounds are used to control and treat infections due to bacteria resistant to existing antibacterials. For example, certain bacteria are resistant to methicillin, and yet others are resistant to vancomycin and/or teicoplanin. The present compounds provide a technique for controlling and treating infections due to such resistant bacterial species.

In carrying out this embodiment of the invention, the compounds of the present invention can be administered by any of the conventional techniques, including the oral route and parenteral routes such as intravenous and intramuscular. The amount of compound to be employed is not critical and will vary depending on the particular compound employed, the route of administration, the severity of the infection, the interval between dosings, and other factors known to those skilled in the art. In general, a dose of from about 0.5 to about 100 mg/kg will be effective; and in many situations, lesser doses of from about 0.5 to about 50 mg/kg will be effective. A compound of the present invention can be administered in a single dose, but in the known manner of antibacterial therapy, a compound of the present invention is typically administered repeatedly over a period of time, such as a matter of days or weeks, to ensure control of the bacterial infection.

Also in accordance with known antibacterial therapy, a compound of the present invention is typically formulated for convenient delivery of the requisite dose. Therefore, in another embodiment, the present invention is directed to a pharmaceutical formulation comprising a compound of the present invention, in combination with a pharmaceutically-acceptable carrier. Such carriers are well known for both oral and parenteral routes of delivery. In general, a formulation will comprise a compound of the present invention in a concentration of from about 0.1 to about 90% by weight, and often from about 1.0 to about 3%. Thus a pharmaceutical composition comprising a compound according to the present invention and a nutritional composition comprising a compound according to the present invention are further embodiments of this invention, both in combination for that purpose with suitable carriers.

Saccharides covalently attached to proteins determine their location or destination in the cell and the metabolic routes they are supposed to follow. Glycoproteins are involved in cell-recognition and thus are involved in signalling processes, in cell-cell interactions, and immunological responses. Thus the compounds of the present invention may be used as diagnostic tools, in vitro and/or in vivo as well as research tools in vitro to study for instance signalling pathways etc. Thus in a further embodiment the present invention concerns a kit of parts that comprises as a research tool and/or diagnostic tool a compound according to the present invention and optionally comprising further reagents, diluents and/or handling instructions.

### EXAMPLES

### Glucopyranosyl-triazolyl-L-alanine

The following conditions are generally applicable to other cycloaddition reaction partners.

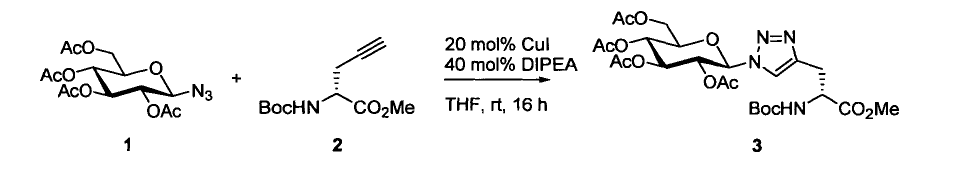

To a solution of tetra-*O*-acetyl-β-D-glucopyranosyl azide (**1**) (36.3 mg, 0.10 mmol) and *N*-Boc-L-propargylglycine methyl ester (**2**) (26.3 mg, 0.12 mmol) in THF (1 mL) was added CuI (3.7 mg, 20 mol%) and DIPEA (6.4 µL, 40 mol%). The mixture was stirred for 16 hours, after which water (1 mL) was added and the product was extracted with DCM (2x). The combined organic layers were washed with a saturated aqueous solution of NaHCO₃ and brine, dried over Na₂SO₄ and concentrated *in vacuo*. The product was purified by flash column chromatography (EtOAc/heptane 1:2) to afford the product as a white solid (51.8 mg, 0.09 mmol, 89%).

In a similar method the following cycloadition reaction partners have been reacted to give the 1,4-triazole glyco amino acids and peptides:

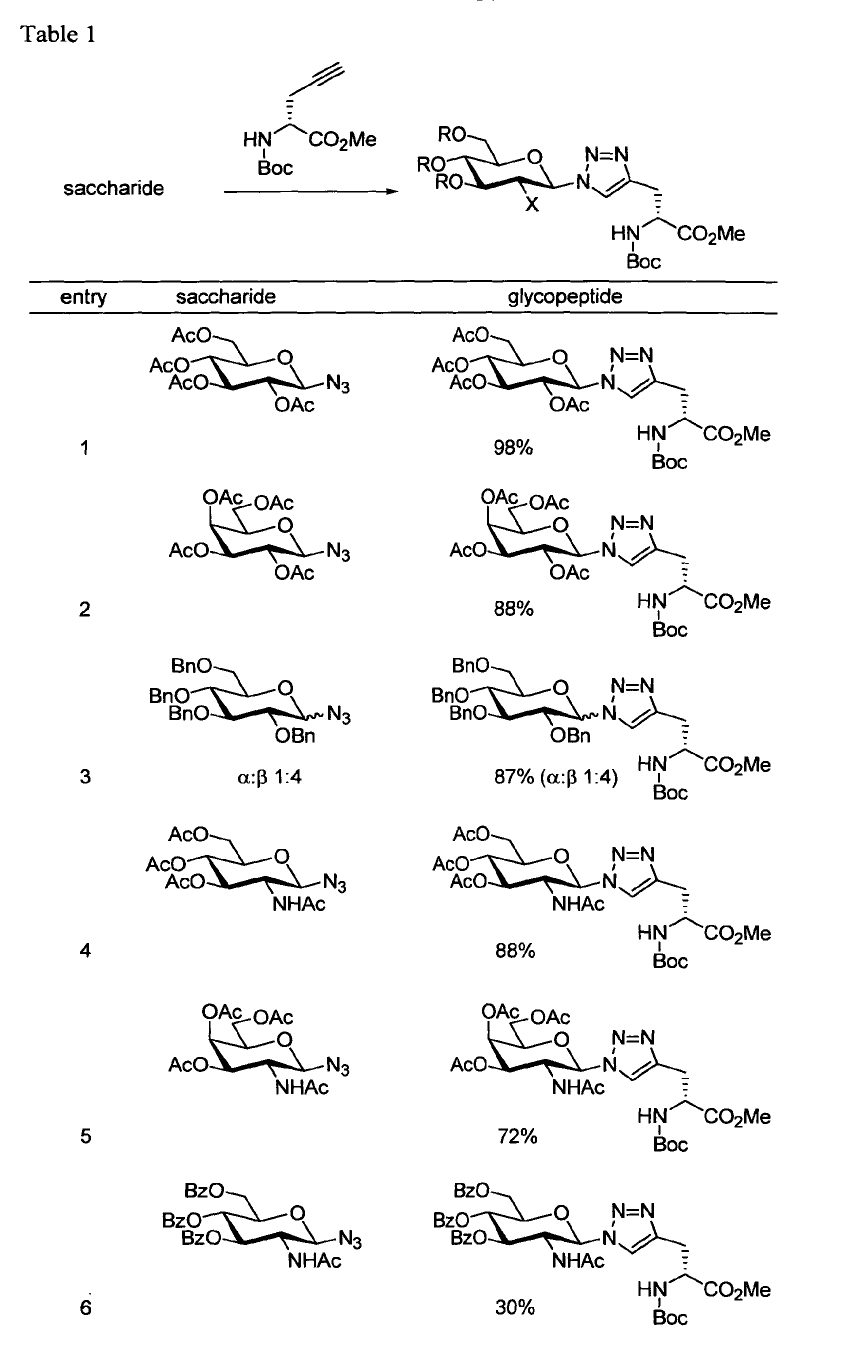

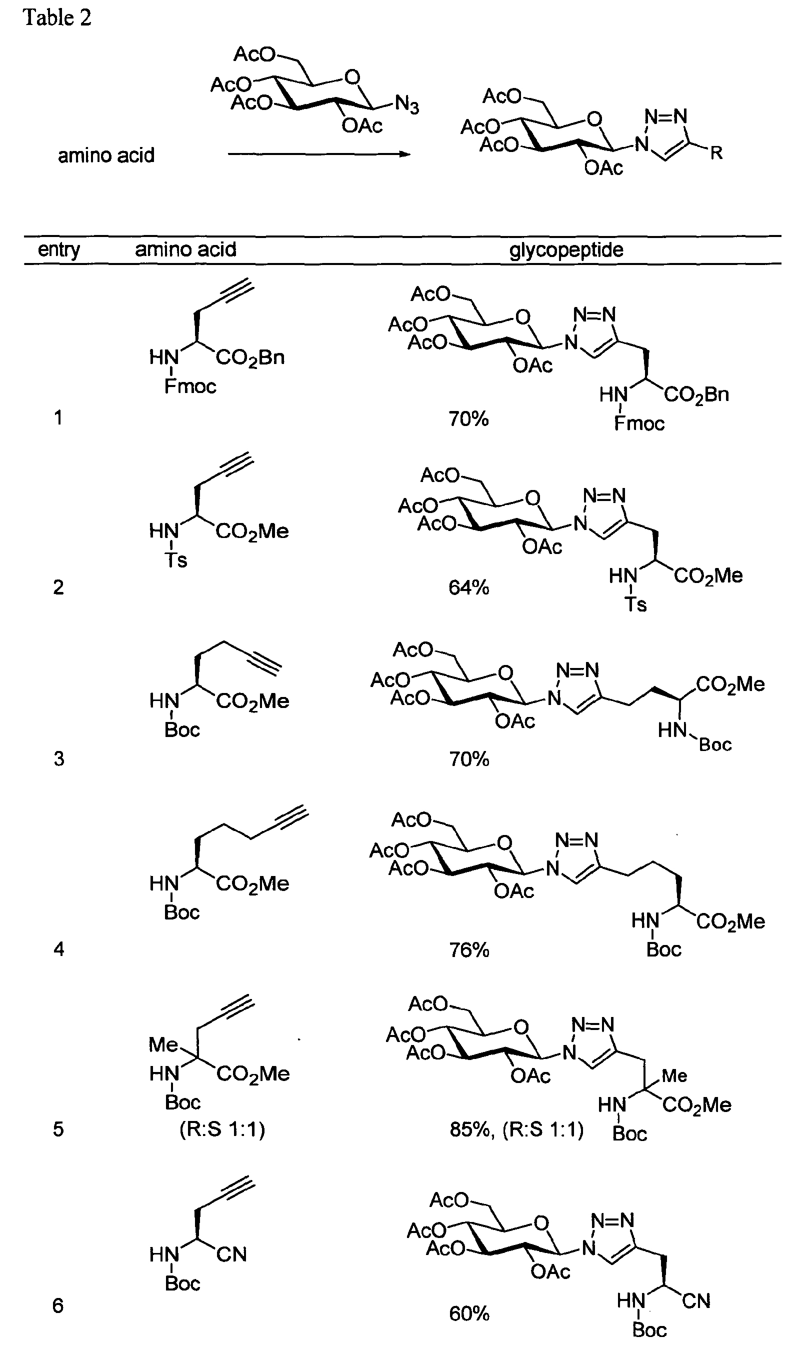

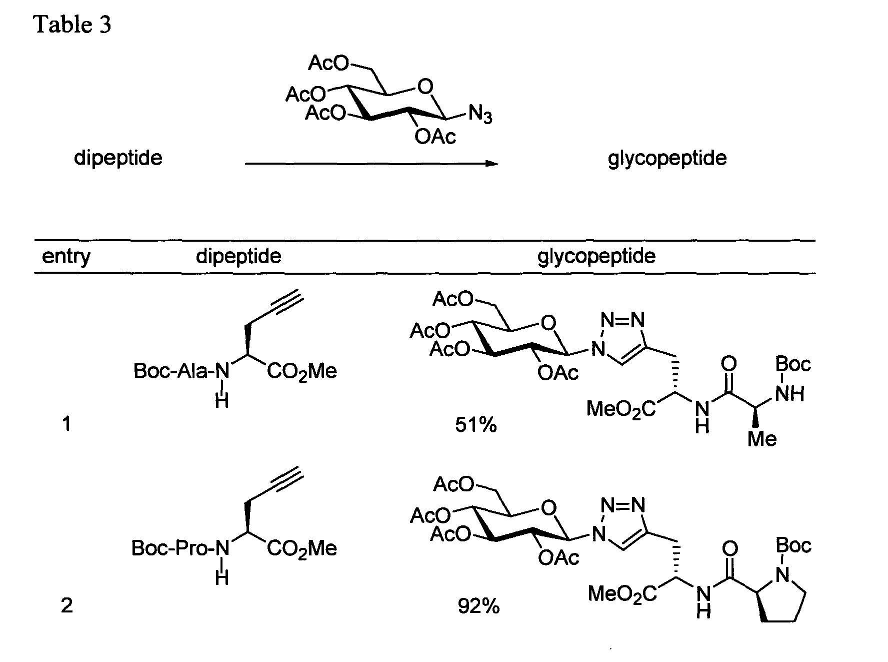

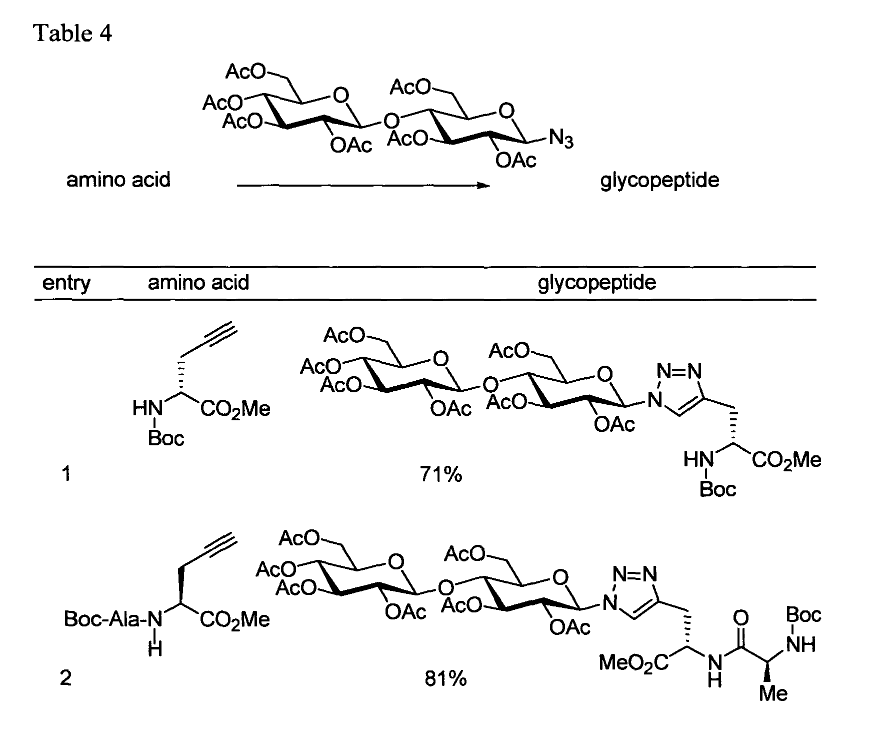

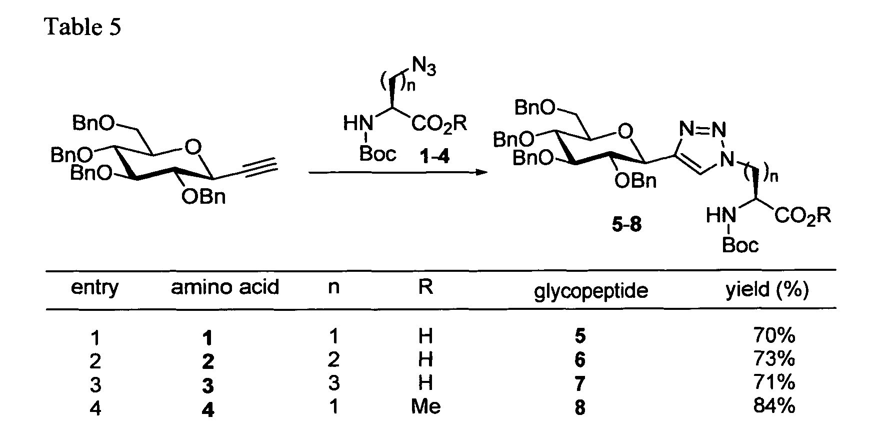

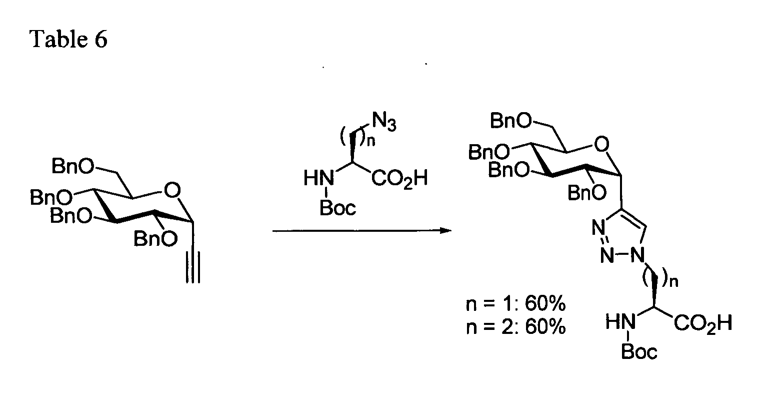

### Synthesis ofpeptide using glyco-amino acids

**First route:** To a solution of *N*-Boc-glycoamino acid (1 equiv) in CH₂Cl₂ (0.1 M) were added H₂N-AA-OMe (1 equiv), DIPEA (1.2 equiv) and EDCI (1.1 equiv) at 0 °C. The reaction mixture was stirred for 16 hours, after which water was added. The product was extracted with DCM (2x). The combined organic layers were washed with a aqueous HCl (0.1 M), a saturated aqueous solution of NaHCO₃ and brine, dried over Na₂SO₄ and concentrated *in vacuo*. The product was purified by flash column chromatography.

**Second route**: An amino acid immobilized on a solid support was suspended in DMF and treated with *N*-Fmoc-glycoamino acid (1.5 equiv), DIPEA (3 equiv) and EDCI (3 equiv) at 0 °C. The reaction mixture was agitated for 16 hours, after which the suspension was filtrated. The resin was washed several times with CH₂Cl₂ and MeOH and dried in vacuo. The resin was suspended in a mixture of CH₂Cl₂ and TFA (1:1) and agitated for 2

### Stability

The glycoamino acid products were subjected to the following reaction conditions, to investigate the stability of the glyco-triazole linkage.
- 2.5 M HCl in EtOAc at rt
- K₂CO₃, MeOH at rt
- 1 M aq. HCl at rt
- 1 M aq. HCl at reflux
- 1 M aq. NaOH at rt
- 1 M HCl in MeOH at reflux

The glyco-triazole linkage appeared to be stable to all these conditions.

## Claims

1. A compound of formula I or I' 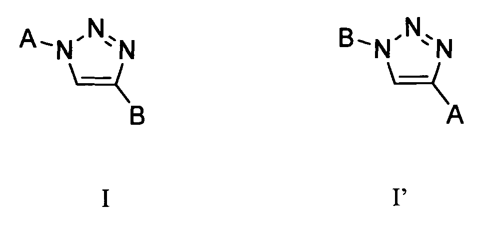 wherein A represents a carbohydrate which is coupled via its anomeric center (I) or parent anomeric center (I') to the triazole ring and B represents an amino acid or a mimetic of an amino acid.

2. A compound of formula II or II' 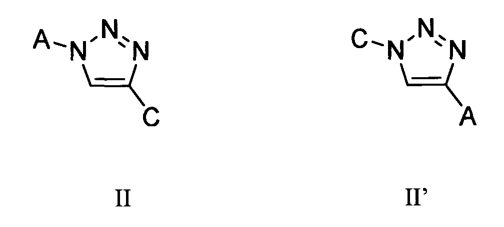 wherein A represents a carbohydrate which is coupled via its anomeric center (I) or parent anomeric center (II') to the triazole ring and C represents a polypeptide or a mimetic of a polypeptide.

3. A compound according to claim 1 or 2 wherein the carbohydrate A comprises 1-20 saccharide units.

4. A compound according to claim 2 or 3 wherein the peptide C or mimetic thereof comprises 2-100 amino acids or mimetic(s) thereof.

5. A compound according to any of the preceding claims wherein B or C is attached to the triazole moiety via the side chain of the amino acid or mimetic thereof or via the side chain of an amino acid or mimetic thereof in the peptide or mimetic thereof.

6. A compound according to any of the preceding claims wherein the carbohydrate is attached to the triazole moiety via the (parent) anomeric carbon atom of a terminal saccharide unit.

7. Method for the preparation of a compound according to claim 1 or 2, comprising the step of the cycloaddition of 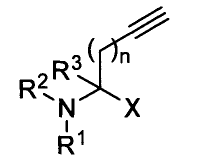 wherein X represents CN, CO₂R⁴, COR⁵, SO₂R⁶, CH₂OR⁷, CH₂NR⁸R⁹, CR¹⁰R¹¹, CONR¹²NR¹³R¹⁴, or X represents the site of attachment to one or more further amino acids;
R¹, R², R³, R⁴, R⁵, R⁶, R⁷, R⁸, R⁹, R¹⁰, R¹¹, R¹², R¹³ and R¹⁴ may be the same or different, and each independently represent H, linear or branched and optionally substituted alkyl, optionally substituted acyl, optionally substituted sulfonyl, optionally substituted aryl, and amine and/or acid protective groups, R¹ and/or R² may also represent the site of attachment to one or more further amino acids,
n = 1-10
with 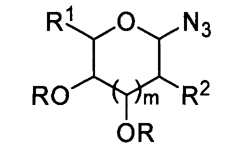 wherein R¹ may be H, CH₂OR or CHORCH₂OR, R² may be H, NHR or OR, R may be the same or different within one molecule and may be H, a saccharide unit or a protective group, m = 0 or 1,
or the cycloaddition of 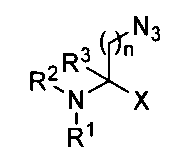 wherein X represents CN, CO₂R⁴, COR⁵, SO₂R⁶, CH₂OR⁷, CH₂NR⁸R⁹, CR¹⁰R¹¹, CONR¹²NR¹³R¹⁴, or X represents the site of attachment to one or more further amino acids;
R¹, R², R³, R⁴, R⁵, R⁶, R⁷, R⁸, R⁹, R¹⁰, R¹¹, R¹², R¹³ and R¹⁴ may be the same or different, and each independently represent H, linear or branched and optionally substituted alkyl, optionally substituted acyl, optionally substituted sulfonyl, optionally substituted aryl, and amine and/or acid protective groups, R¹ and/or R² may also represent the site of attachment to one or more further amino acids, n = 1-10
or 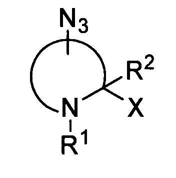 wherein X represents CN, CO₂R³, COR⁴, SO₂R⁵, CH₂OR⁶, CH₂NR⁷R⁸, CR⁹R¹⁰, CONR¹¹NR¹²R¹³ or X represents the site of attachment to one or more further amino acids;
R¹, R², R³, R⁴, R⁵, R⁶, R⁷, R⁸, R⁹, R¹⁰, R¹¹, R¹² and R¹³ may be the same or different, and each independently represent H, linear or branched and optionally substituted alkyl, optionally substituted acyl, optionally substituted sulfonyl, optionally substituted aryl, and amine and/or acid protective groups, R¹ may also represent the site of attachment to one or more further amino acids, the ring may substituted and be saturated or (partly) unsaturated and may be 4-10 membered; the azide can be in any position on the ring.
with 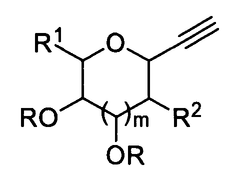 wherein R¹ may be H, CH₂OR or CHORCH₂OR, R² may be H, NHR or OR, R may be the same or different within one molecule and may be H, a saccharide unit or a protective group, m = 0 or 1.

8. Method for the preparation of a compound according to claim 2, said method comprising reacting a compound according to claim 1 as a building block in a peptide synthesis.

9. Method according to claim 8 wherein the peptide synthesis is a peptide synthesis on a solid support, preferably in an automated peptide synthesiser.

10. Method for the preparation of a compound according to claim 2, said method comprising reacting 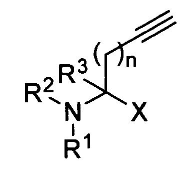 wherein X represents CN, CO₂R⁴, COR⁵, SO₂R⁶, CH₂OR⁷, CH₂NR⁸R⁹, CR¹⁰R¹¹, CONR¹²NR¹³R¹⁴;
R¹, R², R³, R⁴, R⁵, R⁶, R⁷, R⁸, R⁹, R¹⁰, R¹¹, R¹², R¹³ and R¹⁴ may be the same or different, and each independently represent H, linear or branched and optionally substituted alkyl, optionally substituted acyl, optionally substituted sulfonyl, optionally substituted aryl, and amine and/or acid protective groups, n = 1-10
and/or 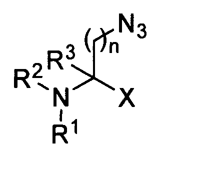 wherein X represents CN, CO₂R⁴, COR⁵, SO₂R⁶, CH₂OR⁷, CH₂NR⁸R⁹, CR¹⁰R¹¹, CONR¹²NR¹³R¹⁴;
R¹, R², R³, R⁴, R⁵, R⁶, R⁷, R⁸, R⁹, R¹⁰, R¹¹, R¹², R¹³ and R¹⁴ may be the same or different, and each independently represent H, linear or branched and optionally substituted alkyl, optionally substituted acyl, optionally substituted sulfonyl, optionally substituted aryl, and amine and/or acid protective groups, n = 1-10
and/or 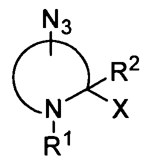 wherein X represents CN, CO₂R³, COR⁴, SO₂R⁵, CH₂OR⁶, CH₂NR⁷R⁸, CR⁹R¹⁰, CONR¹¹NR¹²R¹³;
R¹, R², R³, R⁴, R⁵, R⁶, R⁷, R⁸, R⁹, R¹⁰, R¹¹, R¹² and R¹³ may be the same or different, and each independently represent H, linear or branched and optionally substituted alkyl, optionally substituted acyl, optionally substituted sulfonyl, optionally substituted aryl, and amine and/or acid protective groups, the ring may substituted and be saturated or (partly) unsaturated and may be 4-10 membered; the azide can be in any position on the ring,
as a building block in a peptide synthesis, such as a peptide synthesis on a solid support, preferably in an automated peptide synthesiser.
and reacting azide functional groups in the resulting peptide during peptide synthesis or after peptide synthesis is completed
with 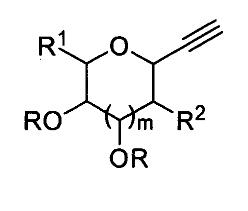 wherein R¹ may be H, CH₂OR or CHORCH₂OR, R² may be H, NHR or OR, R may be the same or different within one molecule and may be H, a saccharide unit or a protective group, m = 0 or 1.
and/or
reacting acetylene functional groups in the resulting peptide during peptide synthesis or after peptide synthesis is completed
with 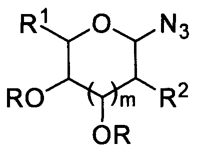 wherein R¹ may be H, CH₂OR or CHORCH₂OR, R² may be H, NHR or OR, R may be the same or different within one molecule and may be H, a saccharide unit or a protective group, m = 0 or 1.

11. Kit of parts comprising as a research tool and/or a diagnostic tool a compound according to any of claims 1-6.
